# EUROPEAN PATENT SPECIFICATION

(11) **EP 0 566 702 B1**
(45) Date of publication and mention of the grant of the patent: **06.03.1996**
(21) Application number: 92916763.3
(22) Date of filing: 24.07.1992
(51) Int. Cl.: A61K 7/06, A61K 7/08

(54) **HAIR CARE SYSTEM**
HAARPFLEGEMITTEL
PROCEDE DE SOIN DES CHEVEUX

(30) Priority: 09.10.1991 CA 2053089
(43) Date of publication of application: 27.10.1993
(73) Proprietor: DE OLIVEIRA, Mariana, Toronto, Ontario M6J 1B4 (CA)
(72) Inventor: DE OLIVEIRA, Mariana, Toronto, Ontario M6J 1B4 (CA)
(74) Representative: Skailes, Humphrey John
(86) International application number: CA9200330
(87) International publication number: WO9306815

(56) References cited:
- EP-A- 0 057 353
- EP-A- 0 123 071
- EP-A- 0 457 565
- WO-A-91/14418
- FR-A- 2 655 544
- US-A- 4 220 167
- US-A- 4 511 555
- US-A- 4 861 593
- US-A- 4 906 460
- US-A- 4 913 898
- SEIFEN,OLE,FETTE. WACHSE JOURNAL 118 (1992) APRIL no. 6, AUGSBURG DE pages 363 - 368 FERD BURMEISTER 'vegetable/plant proteins in shampoos'

## Description

### FIELD OF THE INVENTION

This invention relates to a composition for treating human scalp and hair and particularly relates to a process for enhancing the growth of hair.

### BACKGROUND OF THE INVENTION

The aging process as well as heredity have been long known to contribute to hair loss. In particular, the aging process tends to deplete the production of moisturizing agents such as natural human oils as well as cause the loss of hair growth.

Various attempts have heretofore been employed in order to strengthen hair growth or structure, moisturize the scalp, and minimize the loss of hair growth.

For example, United States Patent No. 321,487 relates to a hair restorative and in particular consists of lime, plumbago, borax, bees wax, tallow, salt and lac sulphur.

Moreover, United States Patent No. 3,168,538 teaches novel compounds and methods of producing sulphinate surface active agents which can be employed in cosmetic preparations and particular in a variety of shampoo formulations due to their high foaming. Yet another shampoo composition is disclosed in United States Patent No. 3,267,039 which consists essentially of treithylonolamine lauryl sulphate in the range from 5% to 25% by weight.

Moreover United States Patent No. 4,946,678 relates to a process of treating human scalp and hair composition comprising the steps of:
(a) applying a sanitizing agent to the balding area of the scalp to open and sanitize the pores and hair follicles of the scalp;
(b) applying heat and a soft agent to soften and separate sebum deposits in the pores and follicles of the scalp once they have been opened by the application of the sanitizing agent;
(c) then rinsing the scalp to remove deposits and agent previously applied thereto; and
(d) then applying chalaza mixture to the scalp to nourish the roots and papillae of the hair in the scalp.

Finally, United States Patent No. 5,017,368 relates to composition for application to hair or scalp which comprises at least one compound selected from the group consisting of forskolin and derivatives thereof and peptide.

These and other compositions have a limited utility.

It is an object of this invention to utilize and improve compositions for treating human scalp and hair compositions.

The broadest aspect of this invention relates to a composition for enhancing the growth of hair comprising a conditioner having the following ingredients based on a total of 500 grams by weight:

| | |
|---|---|
| water | approximately 435.5 gm. |
| cetyl alcohol | approximately 12.5 gm. |
| standamul 100ZK | approximately 12.5 gm. |
| proteins | approximately 35.0 gm. |
| citric acid | approximately 2.5 gm. |
| honey | approximately 0.5 gm. |
| wheat germ oil | approximately 0.5 gm. |
| lecithin | approximately 0.5 gm. |
| herbal extract | approximately 0.5 gm. |
| methylchloroisothiazolinone | approximately 0.5 gm. |

It is another aspect of this invention to comprise a composition consisting of the combination of the conditioner as outlined above plus a shampoo comprising the following ingredients based on a total of 500 grams weight:

| | |
|---|---|
| water | approximately 257.5 gm. |
| sodium lauryl ether sulfate | approximately 200.0 gm. |
| cocamidopropyl betaine | approximately 10.0 gm. |
| cocamide DEA | approximately 10.0 gm. |
| sodium laureth sulfate and a pearlizing agent | approximately 5.0 gm. |
| citric acid | approximately 2.5 gm. |
| proteins | approximately 2.5 gm. |
| honey | approximately 0.5 gm. |
| wheat germ oil | approximately 0.5 gm. |
| lecithin | approximately 0.5 gm. |
| F D & C blue #1 | approximately Q.S. |
| F D & C yellow #5 | approximately Q.S. |
| methylchloroisothiazolinone | approximately 0.5 gm. |
| sodium chloride | approximately 10.0 gm. |

Another aspect of this invention comprises the composition of the combination of the conditioner and shampoo referred to above and a cleansing solution, said cleansing solution comprising the following ingredients in percentage by volume based on the volume of the total composition of said cleansing solution, namely:

| | |
|---|---|
| sodium iodide | approximately 3% |
| potassium iodide | approximately 1.5% |
| sodium thiosulphate | approximately .032% |
| alcohol with water base | 70% alcohol sufficient for the remainder |

Finally, it is another aspect of this invention to provide a process of treating human scalp and hair comprising the steps of:
(a) applying a shampoo to said hair and scalp and massaging the scalp for a period of at least 5 minutes, said shampoo comprising the following ingredients based on a total of 500 grams weight:

| | |
|---|---|
| water | approximately 257.5 gm. |
| sodium lauryl ether sulfate | approximately 200.0 gm. |
| cocamidopropyl betaine | approximately 10.0 gm. |
| cocamide DEA | approximately 10.0 gm. |
| sodium laureth sulfate and a pearlizing agent | approximately 5.0 gm. |
| citric acid | approximately 2.5 gm. |
| proteins | approximately 2.5 gm. |
| honey | approximately 0.5 gm. |
| wheat germ oil | approximately 0.5 gm. |
| lecithin | approximately 0.5 gm. |
| F D & C blue #1 | approximately Q.S. |
| F D & C yellow #5 | approximately Q.S. |
| methylchloroisothiazolinone | approximately 0.5 gm. |
| sodium chloride | approximately 10.0 gm. |

(b) removing said shampoo;
(c) then applying a conditioner and massaging the scalp for a period of at least 5 minutes, such conditioner comprising the following ingredients based on a total of 500 grams weight:

| | |
|---|---|
| water | approximately 435.0 gm. |
| cetyl alcohol | approximately 12.5 gm. |
| standamul 100ZK | approximately 12.5 gm. |
| proteins | approximately 35.0 gm. |
| citric acid | approximately 2.5 gm. |
| honey | approximately 0.5 gm. |
| wheat germ oil | approximately 0.5 gm. |
| lecithin | approximately 0.5 gm. |
| herbal extract | approximately 0.5 gm. |
| methylchloroisothiazolinone | approximately 0.5 gm. |

(d) leaving the conditioner on said hair and scalp with a heating cap to permit the conditioner to penetrate into the pores of the scalp, for a period of approximately 20 minutes;
(e) rinsing said conditioner;
(f) applying a cleansing solution to said scalp for removing deposits of dead skin, said solution comprising the following ingredients in percentage by volume based on the volume of the total composition of said cleansing solution:

| | |
|---|---|
| sodium iodide | approximately 3% |
| potassium iodide | approximately 1.5% |
| sodium thiosulphate | approximately .032% |
| alcohol with water base | 70% alcohol sufficient for the remainder |

(g) leaving the cleansing solution on said scalp.

### DETAILED DESCRIPTION OF THE INVENTION

It has been found that by utilizing a conditioner having the following composition, the hair of an individual tends to grow and thereby retarding the balding process as well as rejuvenating the growth of hair:

| | |
|---|---|
| water | approximately 435.0 gm. |
| cetyl alcohol | approximately 12.5 gm. |
| standamul 100ZK | approximately 12.5 gm. |
| proteins | approximately 35.0 gm. |
| citric acid | approximately 2.5 gm. |
| honey | approximately 0.5 gm. |
| wheat germ oil | approximately 0.5 gm. |
| lecithin | approximately 0.5 gm. |
| herbal extract | approximately 0.5 gm. |
| methylchloroisothiazolinone | approximately 0.5 gm. |

Moreover, the protein utilized in the conditioner comprises of a milk amino acid.

It is the conditioner which apparently encourages the growth of hair.

However, it has also been found that by utilizing the combination of the conditioner as outlined above with shampoo having the following formulation that the growth of hair is further improved, namely:

| | |
|---|---|
| water | approximately 257.5 gm. |
| sodium lauryl ether sulfate | approximately 200.0 gm. |
| cocamidopropyl betaine | approximately 10.0 gm. |
| cocamide DEA | approximately 10.0 gm. |
| sodium laureth sulfate and a pearlizing agent | approximately 5.0 gm. |
| citric acid | approximately 2.5 gm. |
| proteins | approximately 2.5 gm. |
| honey | approximately 0.5 gm. |
| wheat germ oil | approximately 0.5 gm. |
| lecithin | approximately 0.5 gm. |
| F D & C blue #1 | approximately Q.S. |
| F D & C yellow #5 | approximately Q.S. |
| methylchloroisothiazolinone | approximately 0.5 gm. |
| sodium chloride | approximately 10.0 gm. |

It has been found that good results are observed by utilizing glycol disterate and cocamide DEA as the pearlizing agent. Furthermore, the protein used in the shampoo comprises also milk amino acids.

Furthermore, by utilizing the composition and formula of the conditioner and shampoo once a day, good results are observed in the growth of hair in balding areas.

Moreover, it has been found that the hair treatment system described heretofore is somewhat improved by utilizing a cleansing solution having the following ingredients in percentage by volume based on the volume of the total composition of the cleansing solution, namely:

| | |
|---|---|
| sodium iodide | approximately 3% |
| potassium iodide | approximately 1.5% |
| sodium thiosulphate | approximately .032% |
| alcohol with water base | 70% alcohol sufficient for the remainder |

The cleansing solution is utilized to keep the scalp clean and to remove dead skin. Good results are observed by using the cleansing solution three times a day.

The cleansing solution cleans the scalp while the formulation of the conditioner and shampoo moisturizes and enhances the growth of hair.

Moreover the letters Q.S. indicate quantum sufficient or mean "enough".

It has been found that good results are achieved in treating the human scalp and hair by utilizing the compositions referred to above and in particular by the steps of:
(a) applying the shampoo referred to above to the hair and scalp and massaging the scalp and hair for a period of at least 5 minutes;
(b) rinsing the shampoo;
(c) applying the conditioner with the formulation referred to above and massaging the scalp for a period of at least 5 minutes;
(d) leaving the conditioner on said hair and scalp with a heating cap to permit the conditioner to penetrate into the pores of the scalp for a period of approximately 20 minutes;
(e) rinsing the conditioner; and
(f) then applying the cleansing solution referred to above to the scalp for removing deposits of dead skin having the formulation described herein; and
(g) leaving the cleansing solution on said scalp.

## Claims

1. A composition for enhancing the growth of hair comprising a conditioner having the following ingredients based on a total of 500 grams by weight:
| | |
|---|---|
| water | approximately 435.0 gm. |
| cetyl alcohol | approximately 12.5 gm. |
| standamul 100ZK (cetearyl alcohol (and) PEG-40 hydrogenated caster oil (and) stearalkonium chloride) | approximately 12.5 gm. |
| milk amino acids | approximately 35.0 gm. |
| citric acid | approximately 2.5 gm. |
| honey | approximately 0.5 gm. |
| wheat germ oil | approximately 0.5 gm. |
| lecithin | approximately 0.5 gm. |
| herbal extract | approximately 0.5 gm. |
| methylchloroisothiazolinone | approximately 0.5 gm. |

2. A composition as claims in claim 1 whereby said conditioner penetrates into the pores of the scalp.

3. A composition as claimed in claim 1 further including a shampoo acting in combination with said conditioner, said shampoo comprising the following ingredients based on a total weight of 500 grams:
| | |
|---|---|
| water | approximately 257.5 gm. |
| sodium lauryl ether sulfate | approximately 200.0 gm. |
| cocamidopropyl betaine | approximately 10.0 gm. |
| cocamide DEA | approximately 10.0 gm. |
| sodium laureth sulfate and a pearlizing agent | approximately 5.0 gm. |
| citric acid | approximately 2.5 gm. |
| proteins | approximately 2.5 gm. |
| honey | approximately 0.5 gm. |
| wheat germ oil | approximately 0.5 gm. |
| lecithin | approximately 0.5 gm. |
| F D & C blue #1 | approximately Q.S. |
| F D & C yellow #5 | approximately Q.S. |
| methylchloroisothiazolinone | approximately 0.5 gm. |
| sodium chloride | approximately 10.0 gm. |

4. A composition as claimed in claim 3 wherein said protein comprises milk amino acids.

5. A composition as claimed in claim 4 wherein said pearlizing agent comprises glycol distearate and cocamide DEA.

6. A composition as claimed in claim 5 further including a cleansing solution comprising the following ingredients in percentage by volume based on the volume of the cleansing solution:
| | |
|---|---|
| sodium iodide | approximately 3% |
| potassium iodide | approximately 1.5% |
| sodium thiosulphate | approximately .032% |
| alcohol/water base | 70% alcohol Q.S. 100% |

7. A process of treating human scalp and hair comprising the steps of:
(a) applying a shampoo to said hair and scalp and massaging the scalp for a period of at least 5 minutes, such shampoo comprising the following ingredients based on a total of 500 grams weight:
| | |
|---|---|
| water | approximately 257.5 gm. |
| sodium lauryl ether sulfate | approximately 200.0 gm. |
| cocamidopropyl betaine | approximately 10.0 gm. |
| cocamide DEA | approximately 10.0 gm. |
| sodium laureth sulfate and pearlizing agent | approximately 5.0 gm. |
| citric acid | approximately 2.5 gm. |
| proteins | approximately 2.5 gm. |
| honey | approximately 0.5 gm. |
| wheat germ oil | approximately 0.5 gm. |
| lecithin | approximately 0.5 gm. |
| F D & C blue #1 | approximately Q.S. |
| F D & C yellow #5 | approximately Q.S. |
| methylchloroisothiazolinone | approximately 0.5 gm. |
| sodium chloride | approximately 10.0 gm. |
(b) rinsing said shampoo;
(c) then applying a conditioner and massaging the scalp for a period of at least 5 minutes, said conditioner having the following ingredients based on a total of 500 grams by weight:
| | |
|---|---|
| water | approximately 435.5 gm. |
| cetyl alcohol | approximately 12.5 gm. |
| standamul 100ZK (cetearyl alcohol (and) PEG-40 hydrogenated caster oil (and) stearalkonium chloride) | approximately 12.5 gm. |
| proteins | approximately 35.0 gm. |
| citric acid | approximately 2.5 gm. |
| honey | approximately 0.5 gm. |
| wheat germ oil | approximately 0.5 gm. |
| lecithin | approximately 0.5 gm. |
| herbal extract | approximately 0.5 gm. |
| methylchloroisothiazolinone | approximately 0.5 gm. |
(d) leaving the conditioner on said hair and scalp with a heating cap to permit the conditioner to penetrate into the pores of the scalp, for a period of approximately 20 minutes;
(e) rinsing said conditioner;
(f) applying a cleansing solution to said scalp for removing deposit of dead skin, said solution comprising the following ingredients in percentage by volume based on the volume of the total composition of said cleansing solution:
| | |
|---|---|
| sodium iodide | approximately 3% |
| potassium iodide | approximately 1.5% |
| sodium thiosulphate | approximately .032% |
| alcohol with base | 70% alcohol sufficient for the remainder |
(g) leaving the cleansing solution on said human scalp; wherein methods for treatment by surgery or therapy practised on the body are excluded.

8. A process as claimed in claim 7 wherein said pearlizing agent comprises glycol distearate and cocamide DEA.

9. A process as claimed in claim 8 wherein said protein comprises milk amino acids.

10. A process as claimed in claim 9 wherein said cleansing solution is applied three times a day to keep the pores open and clean.

11. A process as claimed in claim 10 wherein said conditioner is applied once a day.

12. A process as claimed in claim 11 wherein said shampoo is applied once a day.

13. A process as claimed in claim 12 wherein said conditioner is absorbed by said hair and said root of said hair.

14. A process as claimed in claim 13, repeated every day.

## Patentansprüche

1. Mittel zur Verstärkung des Haarwuchses, umfassend auf der Grundlage von insgesamt 500 Gramm Gewicht ein die nachstehenden Bestandteile aufweisendes Konditionierungsmittel:
| | |
|---|---|
| Wasser | etwa 435,0 g |
| Cetylalkohol | etwa 12,5 g |
| Standamul 100 ZK (Cetearylalkohol (und) PEG-40 hydriertes Rizinusöl (und) Stearalkoniumchlorid) | etwa 12,5 g |
| Aminosäuren von Milch | etwa 35,0 g |
| Zitronensäure | etwa 2,5 g |
| Honig | etwa 0,5 g |
| Weizenkeimöl | etwa 0,5 g |
| Lecithin | etwa 0,5 g |
| Pflanzenextrakt | etwa 0,5 g |
| Methylchlorisothiazolinon | etwa 0,5 g |

2. Mittel nach Anspruch 1, wobei das Konditionierungsmittel in die Poren der Kopfhaut eindringt.

3. Mittel nach Anspruch 1, das außerdem ein Shampoo enthält, das in Kombination mit dem Konditionierungsmittel wirkt, wobei das Shampoo die nachstehenden Bestandteile, bezogen auf das Gesamtgewicht von 500 g, umfaßt:
| | |
|---|---|
| Wasser | etwa 257,5 g |
| Natriumlaurylethersulfat | etwa 200,0 g |
| Cocamidopropylbetain | etwa 10,0 g |
| Cocamid DEA | etwa 10,0 g |
| Natriumlaurethsulfat und ein Perlmuttglanz erzeugendes Mittel | etwa 5,0 g |
| Zitronensäure | etwa 2,5 g |
| Proteine | etwa 2,5 g |
| Honig | etwa 0,5 g |
| Weizenkeimöl | etwa 0,5 g |
| Lecithin | etwa 0,5 g |
| F D & C Blue #1 | etwa Q.S. |
| F D & C Yellow #5 | etwa Q.S. |
| Methylchlorisothiazolinon | etwa 0,5 g |
| Natriumchlorid | etwa 10,0 g |

4. Mittel nach Anspruch 3, wobei das Protein Aminosäuren von Milch enthält.

5. Mittel nach Anspruch 4, wobei das Perlmuttglanz erzeugende Mittel Glycoldistearat und Cocamid DEA umfaßt.

6. Mittel nach Anspruch 5, das außerdem eine Reinigungslösung einschließt, die die nachstehenden Bestandteile in Volumenprozent, bezogen auf das Volumen der Reinigungslösung, umfaßt:
| | |
|---|---|
| Natriumjodid | etwa 3% |
| Kaliumjodid | etwa 1,5% |
| Natriumthiosulfat | etwa 0,032% |
| Alkohol/Wassergrundlage | 70% Alkohol Q.S. 100% |

7. Verfahren zur Behandlung von Kopfhaut und Haar beim Menschen, umfassend die Schritte:
(a) Anwenden eines Shampoos auf das Haar und die Kopfhaut und Massieren der Kopfhaut für einen Zeitraum von mindestens 5 Minuten, wobei das Shampoo die nachstehenden Bestandteile, bezogen auf das Gesamtgewicht von 500 g, umfaßt:
| | |
|---|---|
| Wasser | etwa 257,5 g |
| Natriumlaurylethersulfat | etwa 200,0 g |
| Cocamidopropylbetain | etwa 10,0 g |
| Cocamid DEA | etwa 10,0 g |
| Natriumlaurethsulfat und Perlmuttglanz erzeugendes Mittel | etwa 5,0 g |
| Zitronensäure | etwa 2,5 g |
| Proteine | etwa 2,5 g |
| Honig | etwa 0,5 g |
| Weizenkeimöl | etwa 0,5 g |
| Lecithin | etwa 0,5 g |
| F D & C Blue #1 | etwa Q.S. |
| F D & C Yellow #5 | etwa Q.S. |
| Methylchlorisothiazolinon | etwa 0,5 g |
| Natriumchlorid | etwa 10,0 g |
(b) Ausspülen des Shampoos;
(c) anschließend Auftragen eines Konditionierungsmittels und Massieren der Kopfhaut für einen Zeitraum von mindestens 5 Minuten, wobei das Konditionierungsmittel die nachstehenden Bestandteile, bezogen auf insgesamt 500 g Gewicht, aufweist:
| | |
|---|---|
| Wasser | etwa 435,5 g |
| Cetylalkohol | etwa 12,5 g |
| Standamul 100 ZK (Cetearylalkohol (und) PEG-40 hydriertes Rizinusöl (und) Stearalkoniumchlorid) | etwa 12,5 g |
| Proteine | etwa 35,0 g |
| Zitronensäure | etwa 2,5 g |
| Honig | etwa 0,5 g |
| Weizenkeimöl | etwa 0,5 g |
| Lecithin | etwa 0,5 g |
| Pflanzenextrakt | etwa 0,5 g |
| Methylchlorisothiazolinon | etwa 0,5 g |
(d) Belassen des Konditionierungsmittels auf dem Haar und der Kopfhaut mit einer beheizten Haube für einen Zeitraum von etwa 20 Minuten, damit das Konditionierungsmittel in die Poren der Kopfhaut eindringt;
(e) Ausspülen des Konditionierungsmittels;
(f) Auftragen einer Reinigungslösung auf die Kopfhaut zur Entfernung von Ablagerungen abgestorbener Haut, wobei die Lösung die nachstehenden Bestandteile in Volumenprozent, bezogen auf das Volumen des gesamten Mittels der Reinigungslösung, umfaßt:
| | |
|---|---|
| Natriumjodid | etwa 3% |
| Kaliumjodid | etwa 1,5% |
| Natriumthiosulfat | etwa 0,032% |
| Alkohol mit Grundlage | 70% Alkohol, ausreichend für den Rest |
(g) Belassen der Reinigungslösung auf der Kopfhaut des Menschen; wobei chirurgische oder therapeutische Behandlungsverfahren, ausgeführt am Körper, ausgeschlossen sind.

8. Verfahren nach Anspruch 7, wobei das Perlmuttglanz erzeugende Mittel Glycoldistearat und Cocamid DEA umfaßt.

9. Verfahren nach Anspruch 8, wobei das Protein Aminosäuren von Milch umfaßt.

10. Verfahren nach Anspruch 9, wobei die Reinigungslösung dreimal täglich angewendet wird, um die Poren offen und sauber zu halten.

11. Verfahren nach Anspruch 10, wobei das Konditionierungsmittel einmal täglich angewendet wird.

12. Verfahren nach Anspruch 11, wobei das Shampoo einmal täglich angewendet wird.

13. Verfahren nach Anspruch 12, wobei das Konditionierungsmittel durch das Haar und die Haarwurzel absorbiert wird.

14. Verfahren nach Anspruch 13, täglich wiederholt.

## Revendications

1. Composition pour favoriser la pousse des cheveux comprenant un conditionneur contenant les ingrédients suivants pour un total de 500 g :
| | |
|---|---|
| eau | approximativement 435,0 g |
| alcool cétylique | approximativement 12,5 g |
| Standamul 100 ZK : (acool cétéarylique (et) PEG-40 huile de ricin hydrogénée (et) chlorure de stéaralkonium) | approximativement 12,5 g |
| acides aminés du lait | approximativement 35,0 g |
| acide citrique | approximativement 2,5 g |
| miel | approximativement 0,5 g |
| huile de germe de blé | approximativement 0,5 g |
| lécithine | approximativement 0,5 g |
| extrait de plantes | approximativement 0,5 g |
| méthylchloroisothiazolinone | approximativement 0,5 g. |

2. Composition selon la revendication 1, ledit conditionneur pénétrant dans les pores du cuir chevelu.

3. Composition selon la revendication 1, comprenant en outre un shampooing agissant en combinaison avec ledit conditionneur, ledit shampooing comprenant les ingrédients suivants pour un poids total de 500 g :
| | |
|---|---|
| eau | approximativement 257,5 g |
| lauryléther sulfate de sodium | approximativement 200,0 g |
| cocamidopropylbétaïne | approximativement 10,0 g |
| cocamide DEA | approximativement 10,0 g |
| laureth sulfate de sodium et un agent conférant un aspect nacré | approximativement 5,0 g |
| acide citrique | approximativement 2,5 g |
| protéines | approximativement 2,5 g |
| miel | approximativement 0,5 g |
| huile de germe de blé | approximativement 0,5 g |
| lécithine | approximativement 0,5 g |
| bleu F D & C # 1 | approximativement q.s.p. |
| jaune F D & C # 5 | approximativement q.s.p. |
| méthylchloroisothiazolinone | approxiamtivement 0,5 g |
| chlorure de sodium | approximativement 10,0 g. |

4. Composition selon la revendication 3, ladite protéine comprenant des acides aminés du lait.

5. Composition selon la revendication 4, ledit agent conférant un aspect nacré comprenant du distéarate de glycol et du cocamide DEA.

6. Composition selon la revendication 5 comprenant en outre une solution de nettoyage comprenant les pourcentages suivants en volume des ingrédients ci-dessous par rapport au volume de la solution de nettoyage :
| | |
|---|---|
| iodure de sodium | approximativement 3 % |
| iodure de potassium | approximativement 1,5 % |
| thiosulfate de sodium | approximativement 0,032 % |
| alcool/base dans l'eau | alcool à 70 % q.s.p. 100 %. |

7. Procédé pour le traitement du cuir chevelu et des cheveux de l'homme comprenant les étapes consistant à :
(a) appliquer un shampooing sur lesdits cheveux et cuir chevelu et masser le cuir chevelu pendant une période d'au moins 5 minutes, un tel shampooing comprenant les ingrédients suivants pour un total de 500 g :
| | |
|---|---|
| eau | approximativement 257.5 g |
| lauryléther sulfate de sodium | approximativement 200,0 g |
| cocamidopropylbétaïne | approximativement 10,0 g |
| cocamide DEA | approximativement 10,0 g |
| laureth sulfate de sodium et agent conférant un aspect nacré | approximativement 5,0 g |
| acide citrique | approximativement 2,5 g |
| protéines | approximativement 2,5 g |
| miel | approximativement 0,5 g |
| huile de germe de blé | approximativement 0,5 g |
| lécithine | approximativement 0,5 g |
| bleu F D & C # 1 | approximativement q.s.p. |
| jaune F D & C # 5 | approximativement q.s.p. |
| méthylchloroisothiazolinone | approximativement 0,5 g |
| chlorure de sodium | approximativement 10,0 g |
b) rincer ledit shampooing;
c) puis appliquer un conditionneur et masser le cuir chevelu pendant une période d'au moins 5 minutes, ledit conditionneur étant constitué des ingrédients suivants pour un total de 500 grammes :
| | |
|---|---|
| eau | approximativement 435,5 g |
| alcool cétylique | approximativement 12,5 g |
| Standamul 100 ZK : (alcool cétéarylique (et) PEG-40 huile de ricin hydrogénée (et) chlorure de stéaralkonium) | approximativement 12,5 g |
| protéines | approximativement 35,0 g |
| acide citrique | approximativement 2,5 g |
| miel | approximativement 0,5 g |
| huile de germe de blé | approximativement 0,5 g |
| lécithine | approximativement 0,5 g |
| extrait de plantes | approximativement 0,5 g |
| méthylchloroisothiazolinone | approximativement 0,5 g; |
d) laisser ledit conditionneur sur lesdits cheveux et cuir chevelu avec une coiffe chauffante de façon à permettre la pénétration du conditionneur dans les pores du cuir chevelu pendant une période d'environ 20 minutes;
e) rincer ledit conditionneur;
f) appliquer une solution de nettoyage audit cuir chevelu de façon à éliminer le dépôt de cellules mortes, ladite solution comprenant les pourcentages en volume suivants des ingrédients ci-dessous par rapport au volume total de ladite solution de nettoyage :
| | |
|---|---|
| iodure de sodium | approximativement 3 % |
| iodure de potassium | approximativement 1,5 % |
| thiosulfate de sodium | approximativement 0,032 % |
| alcool associé à une base | alcool à 70 % q.s.p. |
g) laisser la solution de nettoyage sur ledit cuir chevelu de l'homme;
toute méthode de traitement thérapeutique ou chirurgicale pratiquée sur le corps humain étant exclue.

8. Procédé selon la revendication 7 dans lequel ledit agent conférant un aspect nacré comprend du distéarate de glycol et du cocamide DEA.

9. Procédé selon la revendication 8 dans lequel ladite protéine comprend des acides aminés du lait.

10. Procédé selon la revendication 9 dans lequel ladite solution de nettoyage est appliquée trois fois par jour de façon à conserver les pores ouverts et propres.

11. Procédé selon la revendication 10 dans lequel ledit conditionneur est appliqué une fois par jour.

12. Procédé selon la revendication 11 dans lequel ledit shampooing est appliqué une fois par jour.

13. Procédé selon la revendication 12 dans lequel ledit conditionneur est absorbé par ledit cuir chevelu et les racines desdits cheveux.

14. Procédé selon la revendication 13, répété chaque jour.
